# EUROPEAN PATENT SPECIFICATION

(11) **EP 2 824 112 B1**
(45) Date of publication and mention of the grant of the patent: **21.12.2016**
(21) Application number: 13175985.4
(22) Date of filing: 10.07.2013
(51) Int. Cl.: C07K 16/28, C12N 5/0783

(54) **Method for inducing proliferation of Natural Killer cells by mobile nanomatrices**
Verfahren zur Herbeiführung der Proliferation natürlicher Killerzellen durch mobile Nanomatrices
Procédé pour induire la prolifération de cellules tueuses naturelles par nanomatrices mobiles

(43) Date of publication of application: 14.01.2015
(73) Proprietor: Miltenyi Biotec GmbH, 51429 Bergisch Gladbach (DE)
(72) Inventor: Müller, Sabine, 53797 Lohmar (DE); Scheffold, Alexander, 10711 Berlin (DE); Oerding, Katharina, 41468 Neuss (DE); Huppert, Volker, 51515 Kürten (DE)

(56) References cited:
- EP-A1- 2 711 418
- WO-A2-2009/094273
- ELODIE MACHO FERNANDEZ ET AL: "Activation of invariant Natural Killer T lymphocytes in response to the -galactosylceramide analogue KRN7000 encapsulated in PLGA-based nanoparticles and microparticles", INTERNATIONAL JOURNAL OF PHARMACEUTICS, ELSEVIER BV, NL, vol. 423, no. 1, 28 April 2011 (2011-04-28), pages 45-54, XP028439785, ISSN: 0378-5173, DOI: 10.1016/J.IJPHARM.2011.04.068 [retrieved on 2011-05-07]
- REIM FLORIAN ET AL: "Immunoselection of Breast and Ovarian Cancer Cells with Trastuzumab and Natural Killer Cells: Selective Escape of CD44(high)/CD24(low)/HER2(low) Breast Cancer Stem Cells", CANCER RESEARCH, vol. 69, no. 20, October 2009 (2009-10), pages 8058-8066, XP002717508, ISSN: 0008-5472

## Description

### Field of invention

The present invention relates generally to the field of immunology, in particular to processes for inducing Natural Killer cells to proliferate by nanomatrices.

### Background of the invention

Natural killer (NK) cells are an important part of the innate immune system and are involved in processes against diseases, such as cancer. NK cells are phenotypically defined by the expression of CD56 and the lack of CD3 and T-cell receptor molecules. NK cells can kill target cells without the need for prior sensitization, an effect that is regulated by the balance of stimulatory and inhibitory signals. Importantly, NK cells, different from T cells, do not induce graft-versus-host disease (GvHD) in patients after clinical allogeneic transplantation. These features render NK cells highly promising candidates for clinical applications to combat cancer through adoptive immunotherapy.

WO2004/056392 discloses that soluble anti NK cell receptor antibodies such as NKp30 and NKp46 can induce a slight proliferation of NK cells from fresh human PBMC.

US 5919700 describes a method for inducing proliferation of NK cells by supplementing cell culture media with IL-16. But the IL-16 induced proliferation results in a about five-fold enrichment of natural killer cells only.

US2003/0068306 A1 describes a method for expansion of NK cells from Peripheral Blood Mononuclear cells by using a combination of a special cell culture medium and IL-2 cytokine and OKT-3 antibody. This method relies on autologous feeder T cells that are activated by IL-2 and the anti-CD3 antibody OKT-3 and thus secrete soluble factors to the cell culture medium which induce proliferation in NK cells.

US2004/0115198 A1 describes a method for expanding effector cells including NK cells by crosslinking of the NKG2D receptor with a monoclonal antibody.

US2004/0197903 A1 describes a method for inducing proliferation of NK cells by co-cultivation with cell culture treated dendritic cells. A 10 fold expansion of NK cells is achieved by IL-12, TNFalpha and GM-CSF secreted by the dendritic cells.

Large-scale production of clinical grade NK cells through long-term activation in ex vivo cultures are a means in achieving the aim to improve clinical trial designs by increasing effector to target cell ratios in vivo and by application of superior cytotoxic NK effectors. So far, for NK cells co-cultivation with cell lines and/or feeder cells is part of most proliferation protocols (Suck and Koh, 2012, Hematol Oncol Stem Cell Ther 3: 135-142; Cho and Campana, 2009, Korean J Lab Med, 29: 89-96; EP 1306427A1; US8026097).

Nanosized phosphonate-capped dendrimers showed a slight increase in the number of PBMCs and phenotyping of the cells multiplied in cultures with the dendrimer 3a-G1 revealed the prominence of NK cells. After four weeks in culture, a mean multiplication of the number of NK cells by a factor of 105 was achieved in medium supplemented with Il-2 and 3a-Gl, versus a mean multiplication only by a factor of 7.5 in a medium supplemented with Il-2 alone (Griffe et al, 2007, Angew. Chem, 119:2575-2578).

Although the use of solid phase surfaces such as micron sized magnetic beads are more prominent for T-cell stimulation and expansion, there are some disclosures for NK cell stimulation and expansion as well.

Bryceson et al (2006, Blood, 107: 159-166) showed that responses by resting NK cells, in the absence of exogenous cytokines, can be induced by combinations of activating receptors. Responses measured were Ca2+_ flux triggered by cross-linked mAbs in solution, TNF-α_ and IFN-γ secretion induced by mAbs attached to solid phase surface and cell-sized beads (Dynabeads M-450; Dynal, Oslo, Norway), and cytotoxicity against an FcR+_ target cell in the presence of mAbs directed to NK-cell activation receptors. The combination of NKp46 (CD335) and CD2 was found to be sufficient to activate NK cells in direction to cytokine release and cytotoxicity.

Miltenyi Biotec's NK Cell Activation/Expansion Kit (Miltenyi Biotec GmbH, Bergisch Gladbach, Germany, Order no. 130-094-483) is designed to activate and expand human NK cells. The kit consists of solid phase surface and cell-sized anti-Biotin MACSiBead particles (3,5 µm in size) and biotinylated antibodies against human CD335 and CD2. Anti-Biotin MACSiBead particles loaded with biotinylated antibodies are used to activate and expand resting NK cells purified from human blood or PBMCs.

Contrary to NK cells antibodies against CD3 are a central element in many polyclonal T cell proliferation protocols. Immobilized on a surface, anti-CD3 delivers an activating and proliferation-inducing signal by crosslinking of the T cell receptor complex on the surface of T cells. By immobilizing anti-CD3 and anti-CD28 to simultaneously deliver a signal and a co-stimulatory signal, proliferation can be increased (Baroja et al (1989), Cellular Immunology, 120: 205-217). In WO09429436A1 solid phase surfaces such as culture dishes and beads are used to immobilize the anti-CD3 and anti-CD28 antibodies. Regularly, the immobilization on beads is performed on DynaBeads^{®}M-450 having a size of 4.5 µm in diameter, although US2012/0121649 discloses a method of stimulation and expansion of antigen-specific anti-tumorigenic T cells comprising administering to a subject or to cells in vitro an antigen/MHC/co-stimulatory nanoparticle complex having a size of from about 1 to 100 nm in an amount sufficient to stimulate expansion of an antigen-specific anti-tumorigenic T cell.

In co-pending appl. no. EP12185939.1 is disclosed that polyclonal T cell stimulatory agents such as antibodies, e.g. against CD3 and CD28, attached to nanomatrices, which are characterised by a non-solid surface, can be used to stimulate naive and memory T cells in vitro, although their diameter is smaller than 1 µm, preferentially, smaller than 500 nm, more preferentially smaller than 200 nm. Contrary thereto, it was found that beads with solid surfaces of the same size as the nanomatrices used therein are not able to stimulate T cells at all or to a similar level like the nanomatrices which is in accordance with the well established opinion of the person skilled in the art. Due to their small size the nanomatrices per se, without antibodies attached thereto, do not alter structure, function, activity status or viability of cells, i.e. they do not cause perturbance in the cells and do not interfere with subsequent analyses, experiments and therapeutic applications of the stimulated cells. In addition, preferentially, the nanomatrix is biodegradable and non-toxic to living cells, i.e. the nanomatrix is a biologically inert entity with regard to alterations of the cell function. Therefore the nanomatrix used in the method of EP12185939.1 improves the in-vitro stimulation of T-cells by saving the viability of the cells.

In addition surprisingly, it was found that the polyclonal T cell stimulatory agents such as antibodies, e.g. against CD3 and CD28, attached to nanomatrices may be conjugated to separate nanomatrices (instead of conjugating to the same nanomatrix), which can be mixed hereafter for optimised use. In general, the ratio of nanomatrices to cells is larger than 100:1, preferentially larger than 500:1, most preferentially larger than 1000:1. This results in the possibility of fine-tuning of the nanomatrices used for stimulation of the target T cells, e.g. it facilitates the production process and quality control of the single nanomatrices and improves the flexibility of the reagent, e.g. facilitating the optimisation of the activation conditions for specialised T cell subsets by titrating various CD3 and CD28 concentrations and ratios.

Regardless of EP12185939.1 it was a well established opinion in the scientific community that particles smaller than 1 µm are not convenient to stimulate T cells effectively because such small particles do not provide enough cross-linking to activate T cells (see e.g. US8,012,750B2). It is generally believed that the requirement of cell sized particles is a prerequisite to achieve activation of other cell types such as NK cells and there is no indication in the state of the art that these requirements are different. Especially T cells and NK cells are similar in their signalling molecules and their effector mechanisms such as Perforin/Granzyme and Fas/FasL mediated cytotoxicity as well as induction of cytokine release.

E.g. the publication of Erin R Steenblock and Tarek M Fahmy (Molecular Therapy vol. 16 no. 4, 765-772 April 2008) uses solid-surface nanoparticles (130 nm) and show that these nanoparticle stimulate T cells weaker than microparticles (8 µm). The authors stated that these findings are supported by those of previous reports (Mescher, MF (1992). J Immunol 149: 2402-2405.), demonstrating that micron-sized particles, which are close in size to T cells, provide optimal T-cell stimulation. Mescher's study demonstrated the critical importance of a large, continuous surface contact area for effective CTL activation. Using class I alloantigen immobilized on latex microspheres, particle sizes of 4 to 5 microns were found to provide an optimum stimulus. Below 4 microns, responses decreased rapidly with decreasing particle size, and large numbers of small particles could not compensate for suboptimal size.

Elodie Macho Fernandez et al (2011, International Journal of Pharmaceutics, vol. 423, no. 1, pages 45-54) disclose the use of PLGA-based nanoparticles (PLGA is an amorphous polymer) comprising KRN7000, which is an activator of iNKT cells (which are not NK cells) to activate proliferation of iNKT cells in vitro and in vivo.The activation is indirect as KRN encapsulated into PLGA can be taken up and presented by splenic and liver APC to activate, in a CDld-dependent fashion, iNKT cells in vitro.

WO2009/094273A2 discloses the construction of polymeric aAPCs for the purpose of T cell stimulation/expansion. The particles bear one or multiple functional elements at their surface for the activation and expansion of T cells. The particles can either be microparticles or nanoparticles, but it is indicated that microparticulate aAPCs are more efficient than nanoparticulate aAPCs at stimulating T cell activation. Among the T cells to be stimulated with the aAPC are NKT cells but not NK cells. The only particles used in WO2009094273A2 consist of the amorphous polymer PLGA.

Reim et al (2009, Cancer Research, vol. 69, no. 20, pages 8058-8066) disclose the isolation of NK cells and their expansion using above-mentioned Miltenyi Biotec's NK Cell Activation/Expansion Kit which uses particles of 3,5 µm in size.

Taken together, so far, for NK cells co-cultivation with cell lines and/or feeder cells is part of most proliferation protocols harbouring the risk of contaminating cells. Furthermore cellular compounds are complex and not easy to define exactly and thus may have unpredictable influence on the in vitro culture system in comparison to exactly defined artificial stimulation systems. Beads or microspheres used in the state of the art for NK cell activation via immobilized NK cell stimulatory antibodies are rarely used. In the few existing examples the particles consist of a solid spherical cell-sized particle with activating ligands attached to the surface. Beads of this size have several disadvantages with regard to their potential to interact with NK cells as well as their production, handling and safety in clinical NK cell therapy procedures.
1. Due to the solid surface of the bead the size of interaction area between the bead and cell is limited.
2. Their preparation is complex and costly as compared to soluble antibodies and it is especially inconvenient to generate them in cGMP quality, e.g. due to their size no sterile filtration is possible, sedimentation complicates handling, i.e. constant particle number/volume during filling and antibody loading.
3. They are inconvenient to use for in vitro processes to generate NK cell therapeutics for in vivo use,
   - since they have to be added to cells in defined cell/bead ratios at defined density cell/beads per surface area,
   - titration is inaccurate due to sedimentation,
   - sterile filtration is not possible
   - due to their size they might affect cell viability and function and they cannot simply be removed from cells by centrifugation. Therefore either special protocols for "bead removal" or biodegradable particles have been developed. However both methods suffer from inaccuracies with regard to the actual number of residual beads after the removal process, leaving behind a certain risk for toxic effects if NK cell stimulatory beads are injected into patients. This problem is particularly relevant because of the size of the particles, since each single particle on its own might still have retained the capacity to activate NK cells in vivo.

Therefore, there is a need for an improved in-vitro method for inducing proliferation of NK cells.

### Summary of the invention

Surprisingly, it was found that NK cell stimulatory agents such as antibodies, e.g. against CD2 and CD335 (NKp46), attached to nanomatrices, which are characterised by a mobile polymeric matrix backbone (non-solid surface), which may have embedded within the matrix additional functional compounds, such as magnetic nanocrystalls, can be used to inducing proliferation of NK cells in vitro, although their diameter is smaller than 1 µm, preferentially smaller than 500 nm, more preferentially smaller than 200 nm. Preferentially, the nanomatrices of the invention induce NK cells to proliferate in vitro at least 20-fold, more preferentially at least 50-fold, most preferentially at least 200-fold.

Unexpectedly, these nanomatrices coupled with NK cell stimulatory agents can expand NK cells to a similar scale in vitro as state of the art cell-sized beads also coupled to NK cell stimulatory agents such as e.g. the beads of the Miltenyi Biotec's NK Cell Activation/Expansion Kit (Miltenyi Biotec GmbH, Bergisch Gladbach, Germany, Order no. 130-094-483).

Due to their small size the nanomatrices per se, without antibodies attached thereto, do not alter structure, function, activity status or viability of cells, i.e. they do not cause perturbance in the cells and do not interfere with subsequent analyses, experiments and therapeutic applications of the stimulated cells. In addition, preferentially, the nanomatrix is biodegradable and non-toxic to living cells, i.e. the nanomatrix is a biologically inert entity with regard to alterations of the cell function. Therefore the nanomatrix used in the method of the present invention improves the in-vitro expansion of NK-cells by saving the viability of the cells. In addition as sterile filtration of the small nanomatrices is possible, long term NK cell in vitro expansion under conditions which are compliant with rigorous GMP standards is possible and is a valuable option for clinical application of the in vitro expanded NK cells. These nanomatrices are well suited for use in sterile and closed cell culture system such as described e.g. in WO2009/072003.

In addition surprisingly, it was found that the NK cell stimulatory agents such as antibodies, e.g. against CD2 and CD335, attached to nanomatrices may be conjugated to separate nanomatrices (instead of conjugating to the same nanomatrix), which can be mixed hereafter for optimised use. In general, the ratio of nanomatrices to cells is larger than 100:1, preferentially larger than 200:1, more preferentially larger than 500:1. This results in the possibility of fine-tuning (titration) of the nanomatrices used for inducing proliferation of the target NK cells, e.g. it facilitates the production process and quality control of the single nanomatrices and improves the flexibility of the reagent, e.g. facilitating the optimisation of the activation conditions for specialised NK cell subsets by titrating e.g. various CD2 and CD335 concentrations and ratios.

NK cells are "educated" or "licensed" during their development by contact of the Killer-cell immunoglobulin-like receptor (KIR) molecules on their surface with Major Histocompatibility Complex (MHC) Class I molecules on neighbouring cells. Combinations of KIR molecules are clonally distributed on individual NK cells, resulting in a heterogeneous and continuous level of signalling requirements. Different continuous (in contrast to clearly distinguishable) subsets can be induced to proliferate by titration of the nanomatrices. In addition NK cell subsets are defined based on distinguishable marker expression (e.g. CDl6, CD62L, CD2, CD159a, CD27) which have different signalling requirements for activation.

In comparison thereto, the beads of Miltenyi Biotec's NK Cell Activation/Expansion Kit (Miltenyi Biotec GmgB, Bergisch Gladbach, Germany, Order no. 130-094-483) are used in a bead:cell ratio of 1:2 to 2:1 resulting in a minor flexibility for fine-tuning.

In a first aspect the present invention provides the use of a nanomatrix for inducing NK cells to proliferate in vitro, the nanomatrix comprising
a) a matrix of mobile polymer chains, and
b) attached to said matrix of mobile polymer chains one or more stimulatory agents which provide activation signals to the NK cells;
wherein the nanomatrix is 1 to 500 nm in size, and wherein said mobile polymer chains are of polysaccharides selected from the group consisting of cellulose ethers, starch, gum arabic, agarose, dextran, chitosan, hyaluronic acid, pectins, xanthan, guar gum and alginate.

In a further aspect the present invention provides a method for inducing NK cells to proliferate in vitro, the method comprising contacting a population of NK cells with a nanomatrix, wherein the nanomatrix comprises a matrix of mobile polymer chains, and has attached thereto one or more agents which provide activation signals to the NK cells; thereby activating and inducing the NK cells to proliferate, i.e. to expand; and wherein the nanomatrix is 1 to 500 nm, preferentially 10 to 200 nm, in size, and wherein said mobile polymer chains are of polysaccharides selected from the group consisting of cellulose ethers, starch, gum arabic, agarose, dextran, chitosan, hyaluronic acid, pectins, xanthan, guar gum and alginate. Preferentially, the nanomatrix is biologically inert with regard to alterations of the cell function. In addition preferentially, the nanomatrix is biodegradable.

The stimulated and optionally expanded NK cells achieved with the method of the present invention can be used in subsequent therapeutic or non-therapeutic applications without the need for eliminating or removing the nanomatrix due to the property of the nanomatrix of being biologically inert with regard to alterations of the cell function

Alternatively, due to being soluble or colloidal the nanomatrices can easily be diluted by repeated washing steps to effective concentrations below the NK cell activation threshold after the NK-cell stimulation and proliferation process.

The nanomatrix is 1 to 500 nm, preferentially 10 to 200 nm in size. The nanomatrix is a mobile matrix consisting of a polymeric material but has no solid phase surface in contrast to beads or microspheres. Agents such as anti-CD2 and/or anti-CD335 antibodies which allow for stimulation of NK cells are attached to the mobile polymer chains of the matrix. Within the matrix additional substances, such as magnetic nanocrystalls, fluorescent dyes, etc., can be embedded and add additional functions to the nanomatrix without altering its basic mobile structure, surface features, or cell interaction parameters of the nanomatrix.

This mobility, i.e. flexibility of polymeric matrices in aqueous solutions even when attached to solid surfaces are well known in the field. For example, a quantitative estimation of the flexibility or mobility of dextran and other polymers used for biological or medical applications is given in Bertholon et al. Langmuir 2006, pp45485-5490.

The mobility introduced to the stimulation matrix by the used polymer is well described in multiple publications for polysaccharides and embedded superparamagnetic nanocrystals, which are frequently used for in vivo imaging technologies in clinical situations. These reagents are very similar to the type of reagent which was used here in several of the examples given. It is well known that despite the term "particle" these reagents are actually not comparable to spherical solid surface particles:
"Most of the colloidal (so called) nanoparticles used for medical purpose are coated with polymer or polyelectrolytes and they cannot be really considered as rigid spheres (Di Marco et al. Int J. Nanomed. 2007, p618, line 5 ff,)".

To exemplify directly: this typical feature of polysaccharide embedded superparamagnetic nanocrystalls is best described by the discrepancy in the size values obtained by different methods used for size determination. Typically transmission electron microscopy uses dried samples and thus determines mainly the size of the embedded nanocyrstals, which are always in the range of 10 nm. However, in contrast dynamic laser scattering which also takes into consideration the size of the surrounding matrix in aqueous solution, determines much larger diameters (e.g. 5-10 nm *versus* 80-150nm for AMI-25 a clinical contrast reagent, consisting of a dextran matrix and embedded ironoxide crystals. This means that >99% of the total volume of the complex in aqueous solution is made up by the mobile matrix (Wang et al. Eur. Radiol. 2001: page 2323). For the matrices which were used for example in Example 1, size measurement revealed 30 nm (TEM) and 65 nm (DLS), thus about 80-90 % of the complex in aqueous solution consists of the mobile matrix. Therefore in these types of mobile matrices, the mobile polymer determines the biophysical behaviour during interaction with cells, rather than a stiff particle or solid surface covered, which might in addition be coated with a thin layer of polymer used for attachment. Thus the well-known flexibility or mobility (motility) of the polymer chains in the described matrix is the determining feature and critical difference to solid surface attached antibodies on microspheres classically used for stimulation.

In summary, these examples indicate the current common knowledge about the behaviour of mobile, flexible polymer matrices in aqueous solution (prerequisite for all cell culture applications) and highlight the fact that also the biophysical properties of polymer coated colloids, such as the ironoxide containing polysaccharide matrices used in this study but not limited to those, are determined mainly by the feature of polymer material, independent of the embedded substances.

Therefore the present invention provides an in vitro method for inducing proliferation of NK cells, the method comprising contacting a population of NK cells with a nanomatrix, the nanomatrix comprising
a) a matrix of mobile polymer chains; and
b) attached to said matrix of mobile polymer chains one or more stimulatory agents which provide activation signals to the NK cells; thereby activating and inducing the NK cells to proliferate, wherein the nanomatrix is 1 to 500 nm, preferentially 10 to 200 nm, in size, and wherein said mobile polymer chains are of polysaccharides selected from the group consisting of cellulose ethers, starch, gum arabic, agarose, dextran, chitosan, hyaluronic acid, pectins, xanthan, guar gum and alginate, and wherein the majority (i.e. more than 50%), preferentially more than 80 % and more preferentially more than 90% and most preferentially more than 99% of the total volume of the nanomatrix in aqueous solution consists of mobile polymer chains.

If additional substances, such as magnetic nanocrystalls, fluorescent dyes, etc. are embedded into the matrix, the resulting complex consists mainly of mobile polymer chains, making up more than 50%, preferentially more than 80 % and more preferentially more than 90% and most preferentially more than 99% of the volume in aqueous solution.

### Detailed description of the invention

It was a well established opinion in the scientific community that particles smaller than 1 µm are not convenient to stimulate effectively cells, particularly T cells but also other cells such as NK cells because such small particles do not provide enough cross-linking to activate cells. Therefore, generally, beads or microspheres with solid phase surfaces used to stimulate cells, mostly T cells, are always larger than 1 µm in size in the state of the art, regularly they are cell-sized.

Now unexpectedly, the inventors found that nanomatrices being smaller than 1 µm, preferentially smaller than 500 nm, more preferentially smaller than 200 µm, having a mobile matrix and having attached thereto stimulatory agent(s) are convenient to expand NK cells. It is essential to the present invention that the nanomatrix being smaller than 500 nm has no solid phase surface in contrast to beads or microspheres of the same size. The nanomatrix is like a mesh or net consisting of a mobile polymeric material, preferentially dextran. The nanomatrix is very plastic resulting in the ability to snuggle to the cell surface membrane of target cells, i.e. the NK cells which shall be activated. Therefore, the nanomatrix binds with its agents attached to the mobile matrix to the respective receptors (antigens) on the cell surface, whereby the mobility or flexibility of the matrix allows optimal interaction with the binding partners. To a certain degree the shape of the nanomatrix adapts to the target cell surface thereby extending the contacting surface between nanomatrix and target cell. Due to the size of the nanomatrix of 1 to 500 µm, preferentially 10 to 200 nm, they are too small to cause perturbance in the cell, i.e. the nanomatrix is biologically inert with regard to alterations of the cell function. Such perturbances triggered by direct cell/bead contact is problematic if beads or microspheres of 1 µm or larger in size are used. In addition, preferentially, the nanomatrix is biodegradable and non-toxic to the cells due to the composition consisting of biodegradable polymeric material such as a polymer of dextran. In consequence, the nanomatrix is a completely biologically inert entity with regard to alterations of the cell function but biodegradable. Therefore there is no need to remove the nanomatrix after contacting it with the NK cells for stimulation and proliferation. No disturbing effects occur due to the presence of the nanomatrices in an activated NK cell compositions for subsequent analysis, experiments and/or clinical applications of these cells.

In addition, due to being soluble or colloidal the unbound nanomatrices can easily be diluted by repeated washing steps to effective concentrations below the NK cell activation threshold after the NK-cell expansion process.

The mobile matrix of the nanomatrix has attached thereto one or more stimulatory agents which provide activation signals to the NK cells, thereby activating and inducing the NK cells to proliferate. The agents are molecules which are capable of binding to a cell surface structure and induce the stimulation of the NK cells. One example for agents attached to the mobile matrix of the nanomatrix is anti-CD2 monoclonal antibody (mAb) in combination with an anti-CD335 mAb. Other examples are anti-CD137, anti-CD 134, anti-CD16, anti-CD25, anti-CD52, anti-CD69, anti-CD84, anti-CD122, anti-CD244 (2B4), anti-CD314 (NKG2D), anti-CD336, anti-CD337, anti-NKp80, Notch-ligands, e.g. Delta-like1/4, Jagged1/2 or Cytokine receptors either alone or in various combinations with anti-CD2 or anti-CD335.

Preferentially, the stimulatory agent attached to the nanomatrix is anti-CD2 monoclonal antibody (mAb) in combination with the co-stimulatory protein anti-CD335 mAb. The two antibodies may be coupled to the same nanomatrix or to separate nanomatrices.

Therefore, in one aspect the present invention provides a method for inducing proliferation of NK cells, the method comprising contacting a population of NK cells with a nanomatrix, the nanomatrix comprising
a) a matrix of mobile polymer chains, and
b) attached to said matrix of polymer chains one or more agents which provide activation signals to the NK cells; thereby activating and inducing the NK cells to proliferate;
and wherein the nanomatrix is 1 to 500 nm, preferentially 10 to 200 nm, in size, and wherein said mobile polymer chains are of polysaccharides selected from the group consisting of cellulose ethers, starch, gum arabic, agarose, dextran, chitosan, hyaluronic acid, pectins, xanthan, guar gum and alginate.

The nanomatrix may be biologically inert with regard to alteration of the cell function.

In addition, or alternatively, the nanomatrix may be biodegradable.

The nanomatrix may be of cGMP quality for clinical applications. Sterility can be achieved e.g. by sterile filtration using filters with suitable pore size (200 nm) or by other methods well known by the person skilled in the art. GMP processing of cell products is frequently performed in closed cell culture systems. Use of the nanomatrix in GMP cultivation systems is of advantage due to:
- feature of nanomatrix to pass a sterile filter
- a low particle density or colloidal behaviour that avoids faster sedimentation of matrix compared to NK cells
- dosing of the nanomatrix in relation to the volume of the culture rather than the NK cell number.

The feature of the nanomatrix of being able to pass sterile filters allows the addition to closed cell culture systems being equipped or equipable with sterile filters, e.g. cell cultivation bags (Miltenyi Biotec, Baxter, CellGenics), G-Rex devices (Wilson Wolf manufacturing), WAVE Bioreactors (GE Healthcare), Quantum Cell Expansion System (Terumo BCT), CliniMACS® Prodigy (Miltenyi Biotec, see Apel et al. 2013, Chemie Ingenieur Technik 85:103-110). The nanomatrix can be added to closed cell culture systems using a syringe to push the nanomatrix through the filter or a pump to pull the nanomatrix through the filter from a bag or a vial (connected to a vented vial adapter).

Other agents used to induce NK cell proliferation (cell sized magnetic beads, cell lines) cannot pass a sterile filter. Use of those reagents would require to connect agents to the closed cell culture system using a sterile tubing welder (e.g. Terumo, GE, Genesis). Not all of those tubing welders are designed for operation within a GMP clean room. Thus adding an agent to the closed cell culture system through a sterile filters provides a significant advantage.

Rigid cell sized magnetic particles used to induce NK cell proliferation (Miltenyi Biotec NK cell activation and expansion kit) have a higher density as lymphocytes including NK cells (1.07 g/l) and thus sediment faster than the nanomatrix. Agitated cell culture systems such as the WAVE Bioreactor (GE Healthcare) avoid complete sedimentation of the cells by movement of the container used for cultivation. Movement of the container applies different forces to cells / particles of different size and density, resulting in a relative movement. Induction of proliferation is reduced within the WAVE Bioreactor compared to static culture in cell culture flasks.

The use of the NK cell activation and expansion kit (Miltenyi Biotec) utilizing rigid cell sized magnetic particles has been optimized to be used in a given ratio of beads per cell (e.g. 1:2), requiring determination of the number of NK cells within the culture container. For a clinical application of expanded NK cells this results in an additional sampling step, possibly affected sterility of the product. Use of the nanomatrix of the present invention allows for dosing of the NK cell proliferation inducing agent based on the volume of the culture. The volume of the NK cell containing cell suspension can be determined by a balance or a camera system without affecting the sterility barrier. Dosing of the agent based on volume determination allows for a completely automated cell product manufacturing process (e.g. using the CliniMACS Prodigy® system) without manual cell counting steps.

The contacting can occur e.g. in vitro in any container capable of holding cells, preferably in a sterile environment. Such containers may be e.g. culture flasks, culture bags, bioreactors or any device that can be used to grow cells (e.g. the sample processing system of WO2009072003).

Therefore, in one aspect the present inventions provides a method for inducing NK cells to proliferate in a closed cell culture system, the method comprising contacting a cell culture comprising a population of NK cells within said closed cell culture system with a dosage of nanomatrix, the nanomatrix comprising
a) a matrix of mobile polymer chains; and
b) attached to said matrix of mobile polymer chains one or more stimulatory agents which provide activation signals to the NK cells;
wherein the nanomatrix is 1 to 500 nm in size, and wherein said mobile polymer chains are of polysaccharides selected from the group consisting of cellulose ethers, starch, gum arabic, agarose, dextran, chitosan, hyaluronic acid, pectins, xanthan, guar gum and alginate, and wherein said dosage of nanomatrix is applied sterile to said closed cell culture system, and wherein said dosage depends on the volume of the cell culture in said closed cell culture system.

Said volume of the cell culture can be determined by a balance or a camera system without affecting the sterility barrier.

Determining and applying said dosage may be performed automatically.

The nanomatrix used in the present invention can be a nanomatrix wherein at least one first agent and one second agent are attached to the same mobile matrix. Nanomatrices of this kind are contacted with NK cells, thereby activating and inducing the NK cells to proliferate. The ratio of the first and the second agent attached to the same flexible matrix may be in the range of the ratios of 100:1 to 1:100, preferentially between 10:1 and 1:10, most preferentially between 2:1 and 1:2.

In addition surprisingly, it was found that the nanomatrix used in the present inventive method also can be a nanomatrix wherein at least one first agent and one second agent are attached to separate mobile matrices. A mixture of these nanomatrices is contacted with NK cells, thereby activating and inducing the NK cells to proliferate. The ratio and/or concentration of the mobile matrix having attached thereto the first agent and the mobile matrix having attached thereto the second agent may vary to yield optimal stimulation results depending on the kind of NK cells used and/or agents used. This facilitates the optimisation of the activation conditions for specialised NK cell subsets by titrating various concentrations and ratios of the mobile matrix having attached thereto the first agent and the mobile matrix having attached thereto the second agent. It is advantageous that generally the ratio of nanomatrices to cells is larger than 100:1, preferentially larger than 200:1, more preferentially more than 500:1. The large amount of nanomatrices per cell allows for a fine-tuning of the separate labelled nanomatrices which would be impossible with lower ratios of 1:10 to 10:1 commonly used by stimulation of cells using cell-sized beads.

The nanomatrix used in the present invention is a nanomatrix wherein the mobile matrix consists of a polymeric, preferentially biodegradable material which is non-toxic to cells, i.e. the mobile polymer chains of the nanomatrix are of polysaccharides selected from the group consisting of cellulose ethers, starch, gum arabic, agarose, dextran, chitosan, hyaluronic acid, pectins, xanthan, guar gum and alginate. Preferentially, the nanomatrix used in the present invention is a nanomatrix wherein the mobile matrix consists of a polymer of dextran. Using polymeric material such as dextran for generating the matrix results in a mobile, flexible matrix. The mobility (flexibility or plasticity) of the matrix is superior compared to more rigid particles (having a solid phase surface) or beads of the same size, i.e. nanostructures, for proliferation of cells. In co-pending application EP12185939 it is disclosed that despite their small size nanomatrices (50-200 nm in diameter) comprising a matrix of mobile polymer chains (flexible matrix) indeed have a unique potential to activate T cells when compared to similarly sized particles with a solid surface.

The nanomatrix used in the present invention can be a nanomatrix wherein the mobile matrix is the only or at least main component of the nanomatrix regardless the agents which are attached thereto.

But the nanomatrix used in the present invention also can be a nanomatrix wherein the nanomatrix carries magnetic, paramagnetic, superparamagnetic nano-crystalls, or fluorescent dyes embedded into the mobile matrix, preferentially embedded into the polymer of dextran.

The nanomatrix used in the present invention can be used in a method for proliferating NK-cells wherein the nanomatrix is not removed in subsequent applications of the proliferated NK cells.

Alternatively, the nanomatrix used in the present invention also can be used in a method for proliferating NK-cells wherein the nanomatrix is removed before subsequent applications of the proliferated NK cells.

The herein disclosed inventive method may provide a composition comprising
i) the nanomatrix, the nanomatrix comprising
   a) a matrix of mobile polymer chains; and
   b) attached to said matrix of mobile polymer chains one or more agents which provide activation signals to the NK cells; thereby activating and inducing the NK cells to proliferate; and wherein the nanomatrix is 1 to 500 nm, preferentially 10 to 200 nm, in size, and wherein said mobile polymer chains are of polysaccharides selected from the group consisting of cellulose ethers, starch, gum arabic, agarose, dextran, chitosan, hyaluronic acid, pectins, xanthan, guar gum and alginate,
ii) a population of NK cells which are activated and induced to proliferate triggered by the contact between said nanomatrix and cells.

The nanomatrix may be biologically inert with regard to alteration of the cell function.

The nanomatrix may be biodegradable.

Agents are attached to the same or separate nanomatrices at high density, preferentially with more than 25 µg per mg nanomatrix, more preferentially with more than 50 µg per mg nanomatrix.

This composition may be convenient for generating NK cell therapeutics for in vivo use (pharmaceutical composition). The composition also can be used in other subsequent analyses and experiments.

The herein disclosed inventive method may provide a composition or a pharmaceutical composition comprising a population of stimulated and expanded NK cells. The pharmaceutical composition may comprise a population of proliferated NK cells produced by the method of the present invention, wherein the method is performed in a closed cell culture system such as the sample processing system of WO2009072003.

Nanomatrices can be prepared by various methods known in the art, including solvent evaporation, phase separation, spray-drying, or solvent extraction at low temperature. The process selected should be simple, reproducible and scalable. The resulting nanomatrices should be free-flowing and not aggregate in order to produce a uniform syringeable suspension. The nanomatrix should also be sterile. This can be ensured by e.g. filtration, a terminal sterilization step and/or through aseptic processing. A preparation of nanomatrices is described in Example 1.

### Definitions

The terms "matrix of mobile polymer chains" and "mobile matrix" as used herein have an interchangeable meaning. The term "mobile" refers to the common and well described feature of organic biopolymers such as dextran or others on nanoparticles (see Bertholon et al. Langmuir 2006, pp45485-5490). These polymers consists of mobile (motile), preferentially highly mobile (motile) chains, so the matrix is characterised by the absence of a solid surface as the attachment point for the stimulating agents such as antibodies, and which is in strong contrast to currently used beads or microspheres which regularly have an inflexible, stiff surface. As a result the nanomatrix comprising a matrix of mobile polymer chains is flexible and adjustable to the form of the surface of the cells. In addition as a result the nanomatrix is a nanomatrix wherein the majority (i.e. more than 50%), preferentially more than 80 % and more preferentially more than 90% and most preferentially more than 99% of the total volume of the nanomatrix in aqueous solution consists of mobile polymer chains.

The contact between nanomatrix which has coupled thereto one or more stimulatory agents and the cells to be stimulated benefit from the fact that the nanomatrix has not a fixed, stiff or rigid surface allowing the nanomatrix to snuggle to the cells. The matrix consists of a polymeric, preferentially biodegradable or biocompatible inert material which is non-toxic to cells. The matrix is composed of hydrophilic polymer chains, which obtain maximal mobility in aqueous solution due to hydration of the chains. The mobile matrix is the only or at least main component of the nanomatrix regardless the agents which are attached thereto.

The mobile matrix isof polysaccharides selected from the group consisting of cellulose ethers, starch, gum arabic, agarose, dextran, chitosan, hyaluronic acid, pectins, xanthan, guar gum or alginate. Preferentially the mobile matrix is a polymer of dextran.

The mobile matrix defines the property of the nanomatrix of being very plastic, i.e. flexible, leading to the ability to snuggle to the cell surface membrane of target cells, i.e. the NK cells which shall be activated and proliferated. Therefore, the nanomatrix tightly binds with its agents attached to the mobile matrix to the cells because the mobility of the matrix provides optimal access of the attached ligands or antibodies to their cell surface receptors or antigens. Due to this property the nanomatrix has the ability to provide enough cross-linking to activate NK cells regardless of the small size of the structure, i.e. smaller than 1 µm, preferentially smaller than 500 nm, more preferentially smaller than 200 nm, in size. The adaptability of the nanomatrix caused by the mobile, flexible nanostructure extends the contacting area between cell surface membrane and the nanomatrix resulting in more efficient bindings between cell surface molecules and agents attached to the mobile matrix.

In some embodiments the mobile matrix may embed magnetic, paramagnetic or superparamagnetic nano-crystalls or other substances which add additional functional properties such as fluorescent dyes without altering the basic mobile structure and/or surface features, i.e. interaction with target cells.

The term "agent" which is attached to the mobile matrix of the nanomatrix as used herein refers to molecules which are capable of binding to a cell surface structure and contribute to a stimulation and proliferation of the NK cells. Examples of suitable agents for use in the present invention include agents such as synthesized compounds, nucleic acids and proteins, including polyclonal or monoclonal antibodies, and fragments or derivatives thereof, and bioengineered proteins, such as fusion proteins. In one example, the agents are mitogenic proteins. Mitogenic proteins are two or more proteins that are able to deliver the requisite minimum of two signals to NK-cells in order to cause the NK-cells to become activated. Therefore, in the method of the present invention at least two stimulatory agents are attached to the polymeric flexible matrices (the same or separate matrices) which provide activation signals to the NK cells, preferentially, the at least two agents are selected from the group consisting of antibodies, fragments thereof or ligands to CD2, CD7, CD8a, CD10, CD11a, CD11b, CD11c, CDw12, CD16, CD18, CD25, CD26, CD29, CD30, CD31, CD32c, CD38, CD39, CD43, CD44, CD45, CD45RA, CD45RB, CD45RC, CD45RO, CD46, CD47, CD48, CD49a, CD49b, CD49d, CD49e, CD50, CD52, CD53, CD55, CD56, CD57, CD58, CD59, CD62L, CD63, CD69, CD81, CD82, CD84, CD85C, CD85E, CD85J, CD87, CD94, CD95, CD96, CD97, CD98, CD99, CD100, CD119, CD120a, CD120b, CD122, CD130, CD132, CD147, CD148CD, 158a-CD158k, CD159a, CD159c, CD160, CD161, CD172g, CD178, CD183, CD185, CDw210b, CD212, CD217, CD218a, CD218b, CD220, CD221, CD221, CD222, CD223, CD225, CD226, CD229, CD230, CD232, CD244, CD247, CD257, CD261, CD262, CD263, CD264, CD270, CD274, CD277, CD280, CD295, CD298, CD314, CD316, CD319, CD321, CD328, CD329, CD335, CD336, CD337, CD352, CD354, CD355, CD357, CD360, CD361, CD363. More preferentially, the at least two agents are selected from the group consisting of antibodies, fragments thereof or ligands to CD28, CD137 (4-1BBL), CD226 (DNAM-1), CD314 (NKG2D), CD159c (NKG2C), NKp30, NKp44, CD335 (NKp46), CD69, CD16, NKp80. Most preferentially, the at least two agents are selected from the group consisting of antibodies, fragments thereof or ligands to CD2 and CD335.

Other suitable agents include agents capable of delivering a signal to NK-cells through cytokine receptors such as IL-2R, IL-12R, IL-1R, IL-15R; IFN-gammaR, TNF-alphaR, IL-4R, and IL-10R, including monoclonal antibodies (mAbs) to these receptors, fusion proteins with a reactive end specific for these receptors and the corresponding ligands to these receptors or fractions thereof. Other suitable agents include any agent capable of binding to cellular adhesion molecules on NK cells such as mAbs, fusion proteins and the corresponding ligands or fractions thereof to adhesion molecules in the following categories: cadherins, ICAM, integrins, and selectins. Examples of adhesion molecules on NK-cells are: CD44, CD31, CD18/CD11a (LFA-1), CD29, CD54 (ICAM-1), CD62L (L-selectin), and CD29/CD49d (VLA-4). Other suitable agents include any agents capable of binding to chemokine receptors, including those in the C-C and C-X-C categories. Examples of chemokine receptors associated with NK-cell function include CCR1, CCR2, CCR3, CCR4, CCRS, and CXCR3.

An agent may be attached or coupled to the mobile matrix by a variety of methods known and available in the art. The attachment may be covalent or noncovalent, electrostatic, or hydrophobic and may be accomplished by a variety of attachment means, including for example, chemical, mechanical, enzymatic, or other means whereby an agent is capable of stimulating the cells. For example, the antibody to a cell surface structure first may be attached to the matrix, or avidin or streptavidin may be attached to the matrix for binding to a biotinylated agent. The antibody to the cell surface structure may be attached to the matrix directly or indirectly, e.g. via an anti-isotype antibody. Another example includes using protein A or protein G, or other non-specific antibody binding molecules, attached to matrices to bind an antibody. Alternatively, the agent may be attached to the matrix by chemical means, such as cross-linking to the matrix.

As used herein, the term "antibody" is intended to include polyclonal and monoclonal antibodies, chimeric antibodies, haptens and antibody fragments, and molecules which are antibody equivalents in that they specifically bind to an epitope on the antigen. The term "antibody" includes polyclonal and monoclonal antibodies of any isotype (IgA, IgG, 25 IgE, IgD, IgM), or an antigen-binding portion thereof, including, but not limited to, F(ab) and Fv fragments such as sc Fv, single chain antibodies, chimeric antibodies, humanized antibodies, and a Fab expression library.

The term "biologically inert" as used herein refers to the properties of the nanomatrix, that it is non-toxic to living cells and does not induce strong alterations of the cell function via physical interaction with the cell surface, due to its small size, except the specific ligand/receptor triggering function of the attached ligands or antibodies. The nanomatrices, in addition, may be biodegradable, e.g. degraded by enzymatic activity or cleared by phagocytic cells. The biodegradable material can be derived from natural or synthetic materials that degrade in biological fluids, e.g. cell culture media and blood. The degradation may occur using enzymatic means or may occur without enzymatic means. The biodegradable material degrades within days, weeks or few months, which may depend on the environmental conditions it is exposed to. The biodegradable material should be non-toxic and non-antigenic for living cells and in humans. The degradation products must produce non-toxic by-products. An important aspect in the context of being biologically inert is the fact that the nanomatrix does not induce strong alteration in structure, function, activity status or viability of labelled cells, i.e. it does not cause perturbance of the cells and does not interfere with subsequent experiments and therapeutic applications of the stimulated cells. The mechanical or chemical irritation of the cell is decreased due to the properties of the nanomatrix of being very small, i.e. nano-scale range, and having a mobile matrix which rather snuggles to the cell surface than altering the shape of the cell surface or exerting strong shearing force to the cells, e.g. resulting in membrane rupture.

The term "PBMC" as used herein refers to peripheral blood mononuclear cells isolated from human peripheral blood preparations e.g. by use of a density gradient (e.g. Ficoll, PanColl). "PBMC" consist of lymphocytes and monocytes.

The term "population of NK cells" as used herein refers to a cell suspension containing human natural killer cells. NK cells are positive for CD56 and negative for CD3. In healthy donors NK cells are positive for CD335 (NKp46). A cell suspension containing NK cells can either be peripheral blood, a buffy coat preparation from peripheral blood, cord blood or an apheresis harvest or preparations thereof ("PBMC") or "purified NK cells". A "population of NK cells" may contain several subpopulation of NK cells and NK cells in a different state of differentiation and / or activation.
In addition, the term "population of NK cells" as used herein is not limited to human population of NK cells but also can be of other species such as mouse, rat, cattle, dog, horse, pig and sheep. Thus, the scope of the use of the nanomatrix of the present invention is not limited to human species. For effective stimulation of NK cells of other species than human the nanomatrix of the present invention may be coupled with agents binding to NK cells of other species.

The term "purified NK cells" as used herein refers to cell populations with an increased content of Natural Killer cells. NK cells may be purified by different methods, including FACS® sorting, enrichment by magnetic beads (e.g. CD56 MicroBeads, Miltenyi Biotec), magnetic bead depletion of non-NK cells (e.g. NK cell isolation kit, Miltenyi Biotec), Ficoll based methods (e.g. RosetteSep®, Stem Cell Technologies) or whole blood isolation methods (MACSxpress®, Miltenyi Biotec), as well as panning on antibody coated surfaces.

The term "NK cell subsets" as used herein refers to subpopulations of NK cells that are defined by several markers with populations of CD56+CD3- NK cells expressing this markers at different density, e.g. CD56dim/bright, CD16+/-, CD8+/-, CD2+/-, NKG2A+/-, NKG2C+/- , KIR+/-, CD161+/-, CD25+/-, CD69+/-.

### Embodiments

In one embodiment of the present invention a first nanomatrix of 1 to 500 nm, preferentially 10 to 200 nm in size consists of a mobile matrix of a polymer of dextran and has attached thereto one agent, e.g. anti CD2 mAb. A second nanomatrix of 1 to 500 nm, preferentially 10 to 200 nm in size consists of a mobile matrix of a polymer of dextran and has attached thereto another agent, e.g. anti CD335 mAb. In this case the nanomatrix used in the present invention is a nanomatrix wherein at least one first agent and one second agent are attached to separate mobile matrices.
A mixture of these nanomatrices is contacted with NK cells, thereby activating and inducing the NK cells to proliferate.
Fine-tuning of nanomatrices for the stimulation of the NK cells is easily performed due to the high ratio of nanomatrices to cells (normally larger than 100:1).

In another embodiment of the present invention a nanomatrix of 1 to 500 nm, preferentially 10 to 200 nm in size consists of a mobile matrix of a polymer of dextran and has attached thereto one agent, e.g. anti CD2 mAb. In this case the nanomatrix used in the present invention is a nanomatrix wherein at least one first agent is attached to flexible matrices. This nanomatrix is contacted with NK cells, thereby activating and inducing the NK cells to proliferate.
A second or more (multiple) co-stimulating agents, e.g. anti CD335 mAb, may be added as soluble agents to optimize or support the activation induced by the nanomatrix with the first agent attached thereto.

In another embodiment of the present invention a nanomatrix of 1 to 500 nm, preferentially 10 to 200 nm in size consists of a mobile matrix of a polymer of dextran and has attached thereto two agents which provide activation signals to the cell, e.g. anti CD2 mAb and anti CD335 mAb. In this case the nanomatrix used in the present invention is a nanomatrix wherein at least one first agent and one second agent are attached to the same mobile matrix. Nanomatrices of this kind are contacted with NK cells, thereby activating and inducing the NK cells to proliferate.

In another embodiment of the present invention a nanomatrix of 1 to 500 nm, preferentially 10 to 200 nm in size consists of a mobile matrix of a polymer of dextran and has attached thereto multiple agents which provide activation signals to the cell, e.g. anti-CD2mAb and anti-CD335 mAb or other known activating or co-stimulatory molecules. In this case the nanomatrix used in the present invention is a nanomatrix wherein at least one first agent and multiple other agents are attached to the same mobile matrix.

Nanomatrices of this kind are contacted with NK cells, thereby activating and inducing the NK cells to proliferate.

In another embodiment of the present invention a nanomatrix of 1 to 500 nm, preferentially 10 to 200 nm in size for use in the present invention consists of a mobile matrix of a polymer of dextran and has attached thereto one or more agents which provide activation signals to the cells, e.g. anti CD2 mAb and/or anti CD335 mAb. In addition the nanomatrix carries magnetic, paramagnetic or superparamagnetic nano-crystalls, embedded into the polymer. The nanomatrix is contacted with NK cells, thereby activating and inducing the T cells to proliferate. Optionally, after stimulating of NK cells the unbound magnetic, paramagnetic or superparamagnetic nanomatrix may be removed by applying a magnetic field gradient. Alternatively, the cells labelled with the magnetic nanomatrices may be separated by applying a magnetic field gradient, in particular a high-gradient magnetic field, and subsequent expansion of the purified NK cells.

Although there is no need to remove the nanomatrix after activation and proliferation of the population of NK cells due to its property of being biologically inert with regard to alteration of the cell function one might optionally remove the nanomatrix with mild washing conditions, which are sufficient to wash way the nanomatrices from the cells or cell culture. The nanomatrices can easily be diluted by repeated washing steps to effective concentrations below the NK cell activation threshold. This optionally removing step is much easier performed with the nanomatrices than with beads or microspheres well known in the state of the art due to their small size. If the nanomatrix carries magnetic, paramagnetic or superparamagnetic nano-crystalls, embedded into the polymer than optionally the removal step can be performed by applying a magnetic field gradient to the cell/nanomatrix mixture.

The method of the invention can be used to expand selected NK cell populations for use in treating an infectious disease or cancer. The resulting NK cell population can be genetically transduced and used for immunotherapy or can be used for in vitro analysis of infectious agents such as HIV following expansion of the NK cell population to sufficient numbers, the expanded NK cells are re-infused into the individual or patient. Similarly, a population of tumor-infiltrating lymphocytes can be obtained from an individual afflicted with cancer and the NK cells stimulated to proliferate to sufficient numbers and restored to the individual. In addition, supernatants from cultures of NK cells expanded in accordance with the method of the invention are a rich source of cytokines and can be used to sustain NK cells in vivo or ex vivo.
In another embodiment of the present invention a nanomatrix as described in any preceding embodiment may be used in a closed cell culture system, e.g. the sample processing system of WO2009072003 or cell cultivation bags (Miltenyi Biotec, Baxter, CellGenics), G-Rex devices (Wilson Wolf manufacturing), WAVE Bioreactors (GE Healthcare), Quantum Cell Expansion System (Terumo BCT). The nanomatrices have optimal connectivity to such a closed cell culture system, they can be easily sterile filtrated and integrated into the closed cell culture system. They ease the processes of the closed cell culture system, i.e. stimulation of the NK cells or other target cells, because no removal of the nanomatrices after the stimulation (and expanding) process is necessary as described herein. The nanomatrix can be added in relation to the volume of the culture rather than the NK cell number. Culture volume can be assesses by a balance or a camera system (e.g. in the sample processing system of WO2009072003)
The use of the method of the present invention within a closed cell culture system such as the sample processing system of WO2009072003 results in a safe and easy way to produce a pharmaceutical composition of stimulated NK cells due to the reduced risk of e.g. contaminating agents such as other eukaryotic cells, bacteria or viruses (safer and faster handling by the operator).

In another embodiment of the present invention a nanomatrix as described in any preceding embodiment may be used to induce proliferation in specific subsets of NK cells that are characterized by their unique KIR repertoire. NK cells are "educated" upon their interaction of their KIR molecules with their ligands (i.e. HLA class I molecules). Based on this education process KIR specific NK cell subsets become more cytotoxic and thus more attractive for use as an cellular therapeutic. In this embodiment antibodies binding to KIR molecules are attached to the nanomatrix to mimic the education process in proliferating NK cells.

In another embodiment of the present invention cultivation of NK cells by use of the nanomatrix is performed within a magnetic field. In this embodiment the nanomatrix consists of superparamagnetic cores embedded into the mobile matrix. When applied to a magnetic field magnetic forces are induced in the nanomatrix and ligands triggering activating NK cell receptors can be concentrated for improved induction of proliferation. Cultivation of NK cells within a magnetic field can be performed in a column consisting of a ferromagnetic matrix (e.g. of WO2009072003) or in bag, flask or chamber in close proximity to strong permanent magnet.

In another embodiment of the present invention proliferation of NK cells is induced to achieve an at least 50-fold increase in cell numbers. About 4E8 NK cells can be enriched from a typical apheresis product. The target cell dose for clinical trials is frequently defined as 1-2 E8 NK cells per kg bodyweight of the patient, i.e. 2E10 expanded NK cells for a 100kg patient. The target cell dose can be achieved with a 50-fold expansion (example 3).
The fold expansion is calculated as the NK cell number after the cultivation process divided by the NK cell number that has been used to start the culture.
Typically 30-80% of the NK cells die within the first 5 days of the culture. These are NK cells that are terminally differentiated and functionally exhausted (e.g. not able to proliferate). In case of 80% of the input NK cells dying in the initial cultivation phase a 50-fold expansion as defined above translates to a 250-fold expansion related to the lowest cell number in the culture.

In another embodiment of the present invention proliferation of NK cells is induced to achieve an at least 200-fold increase in cell numbers. About 1E7 NK cells can be enriched from a 100mL donation of whole blood. The target cell dose for clinical trials is frequently defined as 1-2 E8 NK cells per kg bodyweight of the patient, e.g. 2E9 expanded NK cells for a 20kg pediactric patient. The target cell dose can be achieved with a 200-fold expansion (example 3).

In another embodiment of the present invention proliferation of NK cells is induced to achieve an at least 500-fold increase in cell numbers and weekly blood donations (100mL) are used to cultivate NK cells. Within a 8 week weekly treatment 4E10 cultivated NK cells can be given to a cancer patient (example 2).

Prior to stimulation of NK cells by the present invention the NK cells may be directly identified and/or separated or isolated from blood, peripheral mononuclear blood cells (PBMC), body tissue or cells from tissue fluids. The cells are normally identified and/or separated from cell samples from mammals such as humans, mouse, or rat, but especially from humans and preferably from test subject and/or patients. The separation is performed by well known sorting methods in the art. This includes for example affinity chromatography or any other antibody-dependent separation technique known in the art. Any ligand-dependent separation technique known in the art may be used in conjunction with both positive and negative separation techniques that rely on the physical properties of the cells. An especially potent sorting technology is magnetic cell sorting. Methods to separate cells magnetically are commercially available e.g. from Invitrogen, Stemcell Technologies, Cellpro, Advanced Magnetics, or Miltenyi Biotec. In addition to mixtures of NK cells with other cells, such as monocytes, macrophages, dendritic cells, B cells or other cells which are part of hematologic cell samples, such as blood or leukapheresis, highly purified NK cell populations can be used for contacting with the presented invention, including NK cell subpopulations, such as CD56dim NK cells, CD56bright NK cells, CD27 positive and negative subpopulations, CD2 positive and negative subpopulations, CD16 positive and negative subpopulations, KIR positive and negative subpopulations. Nanomatrices provide sufficient crosslinking activity to activating NK cell receptors, therefore additional crosslinking, e.g. via Fc-receptor expressing cells such as monocytes or dendritic cells is not required for activation.
Target cell populations, such as the NK cell populations obtained via the present disclosure may be administered either alone, or as a pharmaceutical composition in combination with diluents and/or with other components such as IL-2 or other cytokines or cell populations. Briefly, pharmaceutical compositions of the present disclosure may comprise a target cell population as described herein, in combination with one or more pharmaceutically or physiologically acceptable carriers, diluents or excipients. A pharmaceutical composition may comprise
a) a population of NK cells, wherein said NK cells are proliferated to therapeutically effective amounts according to the present invention; and b) one or more pharmaceutically or physiologically acceptable carriers, diluents or excipients. Such a composition may contain traces of nanomatrices which are biologically inert with regard to alteration of the cell function but may be biodegradable and which are non-toxic and non-antigenic to humans. Compositions of the present disclosure are preferably formulated for intravenous administration.

Pharmaceutical compositions of the present disclosure may be administered in a manner appropriate to the disease to be treated (or prevented). The quantity and frequency of administration will be determined by such factors as the condition of the patient, and the type and severity of the patient's disease, although appropriate dosages may be determined by clinical trials.

### Examples

### Example 1: Preparation of nanomatrices

Magnetic nanomatrices were produced by a modification of the procedure of Molday and MacKenzie. Ten grams of Dextran T40 (Pharmacia Uppsala, Sweden), 1.5 g FeCl₃ · 6 H₂O and 0.64 g FeCl₂ · 4 H₂O are dissolved in 20 ml H₂O, and heated to 40°C. While stirring, 10 ml 4N NaOH are added slowly and the solution is heated to 70°C for 5 min. The particle suspension is neutralized with acetic acid. To remove aggregates the suspension is centrifuged for 10 min at 2,000 g and filtrated through a 0.22 µm pore-size filter (Millex GV, Millipore, Molsheim, France). Unbound Dextran is removed by washing in a high-gradient magnetic field (HGMF). HGMF washing of magnetic nanomatrices is performed in steelwool columns made as described below and placed in a magnetic field of approx. 0.6 Tesla (MACS® permanent magnet, Miltenyi Biotec GmbH, Bergisch Gladbach, Germany). Ten milliliters of nanomatrix suspension are applied to a 15 x 40 mm column of 2 g steelwool. The loaded column is washed with 30 ml 0.05 M sodium acetate. After removing the column from the external magnetic field, the magnetic nanomatrices are eluted with 0.05 M sodium acetate. The nanomatrices form a brown suspension. The relative particle concentration is given as optical density at 450 nm. The size of the nanomatrices was determined by electron microscopy and dynamic light scattering to be 30 ± 20nm (e.m.) and 65 ± 20 nm (DLS). Dextran content and magnetite content of the matrix within the colloidal solution were determined to be in the range of about 3.5 mg/ml and 4.8 mg/ml, respectively, resulting approximately in a 40:60 w/w ratio. Based on the density of the dried nanomatrix of 2,5g/mL determined by pycnometer and the known densities of dextran and magnetite of 1.6 g/ml and 5.2 g/ml, respectively, the volume of dextran can be calculated to be about 70% for the dried nanomatrix. The nanomatrices show superparamagnetic behavior, as determined by susceptibility measurements. The size of the trapped ferrit microcrystals was determined from magnetic measurements to be approximately 10 nm.

CD2 antibodies (clone LT2, isotype mouse IgG2b) and CD335 (NKp46) antibodies (clone 9E2, isotype mouse IgGl) (Miltenyi Biotec GmbH, Bergisch Gladbach, Germany) were conjugated to the same or separate nanomatrices by standard bioconjugation chemistry (Bioconjugate Techniques, 2nd Edition, By Greg T. Hermanson, Published by Academic Press, Inc., 2008).

### Example 2: Expansion of NK cells from peripheral blood mononuclear cells using nanomatrices at various CD2/CD335 concentrations versus CD2/CD335 MACSiBeads

The current state-of-the-art reagents for activation of highly purified NK cells comprise activating antibodies against CD2/CD335 immobilized on the surfaces of large cell-sized (2-10 µm) particles or co-cultivation with feeder cell lines. Both techniques are error prone and technically difficult to realize and standardize, especially under GMP-compatible production conditions. In contrast nanomatrices can be easily prepared and conveniently be used for cell culture under GMP-conditions. Therefore we compared the potential to induce NK cell proliferation by analysing the expansion potential of the CD2 and CD335 coated nanomatrices at various concentrations with commercially available cell stimulation beads (MACSiBeads, ø 4,5µm, Miltenyi Biotec GmbH). Peripheral blood mononuclear cells (PBMC) were prepared from buffy coat preparations from human whole blood. PBMCs were cultivated in CellGenix CellGro SCGM medium supplemented with 1000 units Interleukin-2 (IL-2) and 5% human AB serum from pre-selected serum lots at 1E6 cells / mL. Magnetic beads were added according to the manufacturer's instructions (1:2 ratio for MACSiBeads) or in volumes of 1, 5, 25 or 50 ul of a 100 ug/ml concentrated CD2/CD335 nanomatrix conjugate (1:1 ratio) per ml medium. As can be seen in Figure 1 and 2 the nanomatrices expand NK cells efficiently even at very low antibody concentrations (100 ng/ml) The expansion at optimal doses (100 ng/ml) was similar or better than the standard reagent (MACSiBeads). At higher doses the expansion was reduced due to overstimulation of the NK cells (activation-induced cell death).

NK cell numbers were increased 3800 fold and 900 fold. NK cell content increased from <10% to 60-90%. Taken together, these results show that CD2/CD335 coated nanomatrices can efficiently induce NK cell proliferation within PBMC at very low antibody concentrations and without the need for additional crosslinking.

### Example 3: Expansion of NK cells from purified NK cells using nanomatrices at various CD2/CD335 concentrations versus CD2/CD335 MACSiBeads

Peripheral blood mononuclear cells (PBMC) were prepared from buffy coat preparations from human whole blood. NK cells were isolated using the NK cell isolation kit (Miltenyi Biotec). Isolated NK cells were cultivated in CellGenix CellGro SCGM medium supplemented with 1000 units Interleukin-2 (IL-2) and 5% human AB serum from pre-selected serum lots at 1E6 NK cells / mL. Magnetic beads were added according to the manufacturer's instructions (1:2 ratio for MACSiBeads) or in volumes of 1, 5, 25 or 50 ul of a 100 ug/ml concentrated CD2/CD335 nanomatrix conjugate (1:1 ratio) per ml medium. The nanomatrices expand NK cells efficiently. Expansion at optimal doses (100 ng/ml) was similar or better than the standard reagent (MACSiBeads). At higher doses the expansion was reduced due to overstimulation of the NK cells (activation-induced cell death).

NK cell numbers were increased 400 fold, 35 fold and 50 fold. Taken together, these results show that CD2/CD335 coated nanomatrices can efficiently induce NK cell proliferation of purified NK cells at very low antibody concentrations and without the need for additional crosslinking.

### Example 4: Expansion of NK cells from purified NK cells using nanomatrices at various CD2/CD335 ratios

Antibodies CD2 (clone LT2) and CD335 (clone 9E2) were conjugated to nanomatrix in ratios of 1:2, 1:1 and 2:1. Purified NK cells were cultivated at 1E6 / mL in CellGenix CellGro SCGM medium supplemented with 1000 units IL-2 and 5% pre-selected human AB serum and the nanomatrix. NK cells were cultivated with the different variants of the nanomatrix for 14 days. Cultures were split when a cell concentration of>5E5 cells was achieved. Expansion rates were 11/21, 18/14 and 13/11 for 1:1, 1:2 and 2:1 ratios (CD2:CD335) at concentrations of 100 and 250 ng/ml. For three additional donors expansion rates were 14/8, 11/8, 13/14 fold and 9/34, 12/18 10/10 fold and 32/25, 23/17, 21/20 fold respectively. References values for use of cell sized beads (NK cell activation and expansion kit, Miltenyi Biotec) were 17/16 (at 100 / 250 ng/mL), 17/15 and 33/37 and 23/18 fold for the same donors.

### Example 5: Expansion of NK cells from peripheral blood mononuclear cells using combinations of nanomatrices conjugated to antibodies CD2 and CD335 (separate nanomatrices)

Antibodies CD2 (clone LT2) and CD335 (clone 9E2) were individually conjugated to nanomatrices (see example 1). Peripheral blood mononuclear cells (PBMC) were prepared from buffy coat preparations from human whole blood. PBMCs were cultivated at 1E6 / mL in CellGenix CellGro SCGM medium supplemented with 1000 units IL-2 and 5% pre-selected human AB serum and 1:1 mixture of the CD2-nanomatrix and CD335 nanomatrix. NK cells were cultivated for 14 and 19 days. Cultures were split when a cell concentration of >5E6 cells was achieved. Expansion rates were 420 / 350 / 290 and 250 fold at 100 / 500 / 2500 / 5000 ng / mL after 14 days and 910 / 750 / 410 and 670 fold after 19 days. NK cells in PBMC cultivated with cell sized beads (NK cell activation and expansion kit, Miltenyi Biotec) expanded 380 (d14) and 530 fold (d19). If similarly effective in large scale a possible 2E10 target cell dose can be obtained with a starting NK cell number of <3E7 NK cells, a cell number that is contained in 100 to 300 mL of whole blood (750 fold expansion assumed)

## Claims

1. The use of a nanomatrix for in-vitro inducing proliferation of NK cells, the nanomatrix comprising
a) a matrix of mobile polymer chains, and
b) attached to said matrix of mobile polymer chains one or more stimulatory agents which provide activation signals to the NK cells;
wherein the nanomatrix is 1 to 500 nm in size, and wherein said mobile polymer chains are of polysaccharides selected from the group consisting of cellulose ethers, starch, gum arabic, agarose, dextran, chitosan, hyaluronic acid, pectins, xanthan, guar gum and alginate.

2. An in vitro method for inducing NK cells to proliferate, the method comprising contacting a population of NK cells with a nanomatrix, the nanomatrix comprising
a) a matrix of mobile polymer chains; and
b) attached to said matrix of mobile polymer chains one or more stimulatory agents which provide activation signals to the NK cells;
wherein the nanomatrix is 1 to 500 nm in size, and wherein said mobile polymer chains are of polysaccharides selected from the group consisting of cellulose ethers, starch, gum arabic, agarose, dextran, chitosan, hyaluronic acid, pectins, xanthan, guar gum and alginate.

3. The method according to claim 2, wherein at least one first and one second stimulatory agents are attached to the same matrix of mobile polymer chains.

4. The method according to claim 2, wherein at least one first and one second stimulatory agents are attached to separate matrices of mobile polymer chains.

5. The method according to claim 4, wherein the ratio of nanomatrices to cells is larger than 100:1 allowing fine-tuning of NK cell proliferation.

6. The method according to any one of claims 2 to 5, wherein one stimulatory agent is an anti-CD2 antibody or fragment thereof.

7. The method according to any one of claims 2 to 6, wherein the second stimulatory agent is an anti-CD335 antibody or fragment thereof.

8. The method according to any one of claims 2 to 7, wherein the matrix of mobile polymer chains consists of a polymer of dextran.

9. The method according to any one of claims 2 to 8, wherein the nanomatrix carries magnetic, paramagnetic or superparamagnetic nano-crystalls, embedded into the matrix of mobile polymer chains.

10. The method according to any one of claims 2 to 9, wherein the stimulatory agent is attached at high density with more than 25 µg per mg nanomatrix.

11. A method for inducing NK cells to proliferate in a closed cell culture system, the method comprising contacting a cell culture comprising a population of NK cells within said closed cell culture system with a dosage of nanomatrix, the nanomatrix comprising
a) a matrix of mobile polymer chains; and
b) attached to said matrix of mobile polymer chains one or more stimulatory agents which provide activation signals to the NK cells;
wherein the nanomatrix is 1 to 500 nm in size, and wherein said mobile polymer chains are of polysaccharides selected from the group consisting of cellulose ethers, starch, gum arabic, agarose, dextran, chitosan, hyaluronic acid, pectins, xanthan, guar gum and alginate and wherein said dosage of nanomatrix is applied sterile to said closed cell culture system, and wherein said dosage depends on the volume of the cell culture in said closed cell culture system.

12. A method according to claim 11, wherein said volume of the cell culture can be determined by a balance or a camera system without affecting the sterility barrier.

13. A method according to claims 11 and 12, wherein determining and applying said dosage are performed automatically.

## Patentansprüche

1. Verwendung einer Nanomatrix zur In-vitro-Herbeiführung der Proliferation von NK-Zellen, wobei die Nanomatrix Folgendes umfasst:
a) eine Matrix von mobilen Polymerketten, und
b) an der Matrix von mobilen Polymerketten hängend, ein oder mehrere stimulierende Agenzien, die Aktivierungssignale an die NK-Zellen bereitstellen;
wobei die Nanomatrix 1 bis 500 nm groß ist, und wobei die mobilen Polymerketten aus Polysacchariden bestehen, die aus der Gruppe ausgewählt werden, die aus Folgendem besteht: Celluloseether, Stärke, Gummiarabikum, Agarose, Dextran, Chitosan, Hyaluronsäure, Pektine, Xanthan, Guaran und Alginat.

2. In-vitro-Verfahren zur Herbeiführung der Proliferation von NK-Zellen, wobei das Verfahren das In-Kontakt-Bringen einer NK-Zellenpopulation mit einer Nanomatrix umfasst, wobei die Nanomatrix Folgendes umfasst:
a) eine Matrix von mobilen Polymerketten; und
b) an der Matrix von mobilen Polymerketten hängend, ein oder mehrere stimulierende Agenzien, die Aktivierungssignale an die NK-Zellen bereitstellen;
wobei die Nanomatrix 1 bis 500 nm groß ist, und wobei die mobilen Polymerketten aus Polysacchariden bestehen, die aus der Gruppe ausgewählt werden, die aus Folgendem besteht: Celluloseether, Stärke, Gummiarabikum, Agarose, Dextran, Chitosan, Hyaluronsäure, Pektine, Xanthan, Guaran und Alginat.

3. Verfahren nach Anspruch 2, wobei mindestens ein erstes und ein zweites stimulierendes Agens an derselben Matrix von mobilen Polymerketten angehängt sind.

4. Verfahren nach Anspruch 2, wobei mindestens ein erstes und ein zweites stimulierendes Agens an unterschiedlichen Matrizen von mobilen Polymerketten angehängt sind.

5. Verfahren nach Anspruch 4, wobei das Verhältnis von Nanomatrizen zu Zellen größer als 100:1 ist, was die Feinabstimmung der Proliferation der NK-Zellen ermöglicht.

6. Verfahren nach einem der Ansprüche 2 bis 5, wobei ein stimulierendes Agens ein anti-CD2 Antikörper oder ein Fragment davon ist.

7. Verfahren nach einem der Ansprüche 2 bis 6, wobei das zweite stimulierende Agens ein anti-CD335 Antikörper oder ein Fragment davon ist.

8. Verfahren nach einem der Ansprüche 2 bis 7, wobei die Matrix von mobilen Polymerketten aus einem Dextranpolymer besteht.

9. Verfahren nach einem der Ansprüche 2 bis 8, wobei die Nanomatrix magnetische, paramagnetische oder superparamagnetische Nanokristalle trägt, eingebettet in die Matrix von mobilen Polymerketten.

10. Verfahren nach einem der Ansprüche 2 bis 9, wobei das stimulierende Agens mit mehr als 25 µg pro mg Nanomatrix bei hoher Dichte anhängt.

11. Verfahren zur Herbeiführung der Proliferation von NK-Zellen in einem geschlossenen Zellkultursystem, wobei das Verfahren das In-Kontakt-Bringen einer Zellkultur, die eine NK-Zellenpopulation innerhalb des geschlossenen Zellkultursystems umfasst, mit einer Dosis Nanomatrix umfasst, wobei die Nanomatrix Folgendes umfasst:
a) eine Matrix von mobilen Polymerketten; und
b) an der Matrix von mobilen Polymerketten hängend, ein oder mehrere stimulierende Agenzien, die Aktivierungssignale an die NK-Zellen bereitstellen;
wobei die Nanomatrix 1 bis 500 nm groß ist, und wobei die mobilen Polymerketten aus Polysacchariden bestehen, die aus der Gruppe ausgewählt werden, die aus Folgendem besteht: Celluloseether, Stärke, Gummiarabikum, Agarose, Dextran, Chitosan, Hyaluronsäure, Pektine, Xanthan, Guaran und Alginat, und wobei die Dosis Nanomatrix steril auf das geschlossene Zellkultursystem angewendet wird, und wobei die Dosis vom Volumen der Zellkultur im geschlossenen Zellkultursystem abhängt.

12. Verfahren nach Anspruch 11, wobei das Volumen der Zellkultur mittels einer Waage oder eines Kamerasystems bestimmt werden kann, ohne die Sterilitätsgrenze zu beeinflussen.

13. Verfahren nach den Ansprüchen 11 und 12, wobei das Bestimmen und Anwenden der Dosis automatisch durchgeführt wird.

## Revendications

1. Utilisation d'une nanomatrice in vitro pour induire la prolifération des cellules NK, la nanomatrice comprenant
a) une matrice de chaînes polymères mobiles, et
b) attachés à ladite matrice de chaînes polymères mobiles, un ou plusieurs agents stimulateurs qui fournissent des signaux d'activation aux cellules NK;
où la dimension de la nanomatrice est comprise entre 1 et 500 nm, et où lesdites chaînes polymères mobiles sont des polysaccharides choisis dans le groupe comprenant les éthers de cellulose, l'amidon, la gomme arabique, l'agarose, le dextrane, le chitosane, l'acide hyaluronique, les pectines, le xanthane, la gomme guar et l'alginate.

2. Procédé in vitro pour induire des cellules NK à proliférer, le procédé comprenant la mise en contact d'une population de cellules NK avec une nanomatrice, la nanomatrice comprenant
a) une matrice de chaînes polymères mobiles; et
b) attachés à ladite matrice de chaînes polymères mobiles, un ou plusieurs agents stimulateurs qui fournissent des signaux d'activation aux cellules NK;
où la dimension de la nanomatrice est comprise entre 1 et 500 nm, et où lesdites chaînes polymères mobiles sont des polysaccharides choisis dans le groupe comprenant les éthers de cellulose, l'amidon, la gomme arabique, l'agarose, le dextrane, le chitosane, l'acide hyaluronique, les pectines, le xanthane, la gomme guar et l'alginate.

3. Procédé selon la revendication 2, où ledit au moins un premier agent stimulateur et ledit un deuxième agent stimulateur sont fixés à la même matrice de chaînes polymères mobiles.

4. Procédé selon la revendication 2, où ledit au moins un premier et un deuxième agent stimulateur sont fixés à des matrices distinctes de chaînes polymères mobiles.

5. Procédé selon la revendication 4, où le ratio des nanomatrices aux cellules est supérieur à 100:1 permettant le réglage fin de la prolifération des cellules NK.

6. Procédé selon l'une quelconque des revendications 2 à 5, où un agent stimulateur est un anticorps anti-CD2 ou un fragment de celui-ci.

7. Procédé selon l'une quelconque des revendications 2 à 6, où le deuxième agent stimulateur est un anticorps anti-CD335 ou un fragment de celui-ci.

8. Procédé selon l'une quelconque des revendications 2 à 7, où la matrice de chaînes polymères mobiles est constituée d'un polymère de dextrane.

9. Procédé selon l'une quelconque des revendications 2 à 8, où la nanomatrice porte des nanocristaux magnétiques, paramagnétiques ou superparamagnétiques, noyés dans la matrice de chaînes polymères mobiles.

10. Procédé selon l'une quelconque des revendications 2 à 9, où l'agent stimulateur est fixé avec une densité élevée de plus de 25 µg par mg de nanomatrice.

11. Procédé pour induire des cellules NK à proliférer dans un système de culture cellulaire fermé, le procédé comprenant la mise en contact d'une culture cellulaire comprenant une population de cellules NK à l'intérieur dudit système de culture cellulaire fermé avec un dosage de nanomatrice, la nanomatrice comprenant
a) une matrice de chaînes polymères mobiles; et
b) attachés à ladite matrice de chaînes polymères mobiles, un ou plusieurs agents stimulateurs qui fournissent des signaux d'activation aux cellules NK;
où la dimension de la nanomatrice est comprise entre 1 et 500 nm, et où lesdites chaînes polymères mobiles sont des polysaccharides choisis dans le groupe comprenant les éthers de cellulose, l'amidon, la gomme arabique, l'agarose, le dextrane, le chitosane, l'acide hyaluronique, les pectines, le xanthane, la gomme guar et l'alginate et où ledit dosage de nanomatrice est appliqué à l'état stérile audit système de culture cellulaire fermé, et où ledit dosage dépend du volume de la culture cellulaire dans ledit système de culture cellulaire fermé.

12. Procédé selon la revendication 11, où ledit volume de la culture cellulaire peut être déterminé par un contrepoids ou système de caméra sans affecter la barrière de stérilité.

13. Procédé selon les revendications 11 et 12, où la détermination et l'application dudit dosage sont effectuées automatiquement.
